# EUROPEAN PATENT SPECIFICATION

(11) **EP 4 470 545 B1**
(45) Date of publication and mention of the grant of the patent: **22.10.2025**
(21) Application number: 23176265.9
(22) Date of filing: 31.05.2023
(51) Int. Cl.: A61K 35/12, A61K 9/06, A61K 8/92, A61P 17/00, A61K 8/64, A61K 35/35, A61K 8/44

(54) **CELL-FREE LIPOASPIRATE-DERIVED PREPARATION, COMPOSITIONS COMPRISING THE PREPARATION AND USES THEREOF**
ZELLFREIE LIPOASPIRATABGELEITETE ZUBEREITUNG, ZUSAMMENSETZUNGEN MIT DER ZUBEREITUNG UND VERWENDUNGEN DAVON
PRÉPARATION DÉRIVÉE DE LIPOASPIRAT SANS CELLULES, COMPOSITIONS COMPRENANT LA PRÉPARATION ET UTILISATIONS ASSOCIÉES

(43) Date of publication of application: 04.12.2024
(73) Proprietor: Linio Biotech Ltd, 00150 Helsinki (FI)
(72) Inventor: UUSMIES, Jertta-Riina, 00180 Helsinki (FI); REIJONSAARI, Karita, 00180 Helsinki (FI); SANMARK, Enni, 00180 Helsinki (FI); YLIKOMI, Timo, 00180 Helsinki (FI)
(74) Representative: Primrose Oy

(56) References cited:
- WO-A2-2011/019822
- US-B2- 9 631 176
- YU ZIYOU ET AL: "Fat extract promotes angiogenesis in a murine model of limb ischemia: a novel cell-free therapeutic strategy", vol. 9, no. 1, 8 November 2018 (2018-11-08), pages 1 - 14, XP055780341, Retrieved from the Internet <URL:http://link.springer.com/content/pdf/10.1186/s13287-018-1014-y.pdf> DOI: 10.1186/s13287-018-1014-y
- HE YUNFAN ET AL: "Human adipose liquid extract induces angiogenesis and adipogenesis: a novel cell-free therapeutic agent", vol. 10, no. 1, 14 August 2019 (2019-08-14), pages 1 - 14, XP055929308, Retrieved from the Internet <URL:http://link.springer.com/article/10.1186/s13287-019-1356-0/fulltext.html> DOI: 10.1186/s13287-019-1356-0
- XU YUDA ET AL: "Cell-Free Fat Extract Increases Dermal Thickness by Enhancing Angiogenesis and Extracellular Matrix Production in Nude Mice", AESTHETIC SURGERY JOURNAL, vol. 40, no. 8, 13 July 2020 (2020-07-13), US, pages 904 - 913, XP055879003, ISSN: 1090-820X, DOI: 10.1093/asj/sjz306
- ZHAOYANG CHEN ET AL: "Human decellularized adipose matrix derived hydrogel assists mesenchymal stem cells delivery and accelerates chronic wound healing", JOURNAL OF BIOMEDICAL MATERIALS RESEARCH PART A, JOHN WILEY & SONS, US, vol. 109, no. 8, 9 December 2020 (2020-12-09), pages 1418 - 1428, XP072311105, ISSN: 1549-3296, DOI: 10.1002/JBM.A.37133

## Description

### TECHNICAL FIELD

The invention belongs to the fields of medicine and cosmetics, in particular to a cell-free lipoaspirate-derived preparation, compositions comprising the same and uses thereof for therapeutic and/or cosmetic treatments of the skin.

### BACKGROUND

The human skin is the outer covering of the body acting as an important protective barrier against sunlight, heat, pathogens, chemicals, injuries and other external factors. Sometimes life renders the skin damaged, aesthetically displeasing and functionally compromised. Over 100 million patients acquire scars from surgeries yearly, and millions suffer from chronic skin conditions and traumatic skin defects. In addition, the skin is affected by aging and environmental factors. This can lead to restricted lifestyles, lower quality of life and mental distress.

Skin regeneration, a natural skin renewal process, is essential to maintaining the health and vitality of the skin, as well as to healing of traumatic and other skin defects.

Although some skin regeneration promoting techniques and products have been developed, their effects are still unsatisfactory. Therefore, there remains a well-recognized need for allogenic products that can effectively and safely enhance skin regeneration.

### SUMMARY

In one aspect, the present invention provides a cell-free lipoaspirate-derived preparation which is sterile-filtered and comprises at least one of a lipid fraction and an aqueous fraction of a lipoaspirate. In other words, the preparation is free of an adipose tissue fraction of a lipoaspirate, which tissue fraction comprises adipocytes and other cell naturally present in a human adipose tissue.

In another aspect, the present invention provides a cosmetic and a pharmaceutical composition comprising the cell-free preparation of the invention and a physiologically acceptable carrier, adjuvant and/or excipient.

Also provided is use of the present cell-free preparation and the cosmetic composition for cosmetically treating the skin.

In a further aspect, the invention provides a non-therapeutic method for cosmetically treating the skin, the method comprising: applying the cell-free preparation or the cosmetic preparation of the invention to the skin.

In a still further, the invention provides the cell-free preparation or the pharmaceutical composition of the invention for use in treating a clinical skin condition.

In an even further aspect, the present invention provides a method of manufacturing a cosmetic composition for a non-therapeutic treatment of skin comprising the cell-free preparation of the invention.

Further aspects, embodiments and details are set forth in following figures, detailed description, examples, and dependent claims.

### BRIEF DESCRIPTION OF THE DRAWINGS

The accompanying drawings illustrate several embodiments of the disclosed subject matter, and together with the description, serve to explain principles of the disclosed compositions and methods.
Figure 1 shows photographs of right and left profiles of a male whose temple and cheek areas with acne scars were treated with laser resurfacing with (on the right) or without (on the left) topical administration of the present cell-free lipoaspirate-derived preparation. The photographs illustrate the healing and re-epithelization process 2 days after the treatment.
Figure 2 shows photographs of a four times operated leg with a three years old scar treated with laser surfacing, taken at different time points (6h, 2d, 28d) after the treatment. The lower half of the scar was treated twice by topical administration of the present cell-free lipoaspirate-derived preparation.
Figure 3 shows photographs of a skin area with some scars before (on the left) and four weeks after (on the right) a microneedling treatment followed by topical administration of the cell-free lipoaspirate-derived preparation of the invention.
Figure 4 shows photographs of the eye and mouth areas before (on the left) and four weeks after (on the right) mesotherapy with the cell-free lipoaspirate-derived preparation of the invention.

### DETAILED DESCRIPTION

The following detailed description illustrates aspects and embodiments of the present invention and ways in which they can be implemented. Although some modes of carrying out the present invention have been disclosed, it is to be understood that other embodiments for carrying out or practicing the present invention are also possible. In other words, the scope of the present invention will be limited only by the appended claims.

It is also to be understood that the terminology used herein has the same meaning as commonly understood by one of ordinary skill in the art to which this invention belongs, unless otherwise specified.

As used herein, a singular noun, unless otherwise specified, carries also the meaning of the corresponding plural noun. In other words, the singular expressions "a", "an" and "the" carries not only the meaning of "one" but also "one or more", unless otherwise specified.

The term "and/or" in a phase such as "X and/or Y" shall be understood to mean either "X and Y" or "X or Y" and shall be taken to provide explicit support for both meanings or for either meaning.

The terms "comprising", "including", "containing" and "having" can be used interchangeably, and are intended to be construed in a non-exclusive manner, namely allowing for items, components or elements not explicitly described also to be present.

In one aspect, the present invention provides a cell-free preparation comprising or consists of a lipid fraction and/or an aqueous fraction of a lipoaspirate.

As used herein, the term "cell-free" refers to a preparation that is devoid or essentially devoid of cells. Techniques for determining whether a given preparation is cell-free or not are readily available in the art.

As used herein, the term "lipoaspirate" refers to material obtainable by liposuction.

Liposuction is a surgical removal of subcutaneous adipose tissue (i.e., hypodermal adipose tissue) by means of aspiration cannulas introduced through small skin incisions, assisted by suction. Various liposuction techniques are readily available and have evolved as technology has improved. Technological advancements added to the traditional dry liposuction technique include ultrasonic liposuction, water-assisted liposuction, and radiofrequency-assisted liposuction to name some non-limiting examples. New state-of-the-art liposuction technique is water-assisted liposuction, a technique that utilizes a pressurized stream of saline/aqueous buffered solution in an amount that is about two to three times larger than the volume of subcutaneous fat to be removed, thereby causing the fatty subcutaneous tissue layer to swell and become firm prior to aspiration and to ease the removal of subcutaneous fat/tissue from different areas in the body. Commonly used saline/aqueous buffered solution in water-assisted liposuction is called "tumescent solution". Accordingly, the liposuction technique can be called tumescent liposuction. Traditional tumescent solution contains local anesthetics such as lidocaine and adrenaline or epinephrine. Notably, the present invention is not limited to any particular liposuction technique, as the choice of the technique often depends on patient characteristics and surgeon preference.

As used herein, the term "liposuction solution" refers to any physiologically acceptable aqueous solution suitable for use as an infiltrate in any liposuction technique, including not only the conventional tumescent liposuction but also other water-assisted liposuction techniques. Non-limiting examples of suitable liposuction solutions include 0.9% sodium chloride (saline), Ringer Acetat, Ringer fundin, Ringer lactate and Normofundin. Those skilled in the art can easily select an appropriate aqueous buffered solution for each case. In some embodiments, the liposuction solution may contain any approved local anesthetic, such as lidocaine or prilocaine, typically 0.5% or 1% lidocaine (up to 55 mg/kg). In some other embodiments, the liposuction solution may contain adrenaline/epinephrine, typically 1% adrenaline (up to 7mg/kg), for temporary vasoconstriction to prevent bleeding during liposuction. In some embodiments, the liposuction solution may contain both lidocaine (and/or any other appropriate local anesthetic) and adrenaline, preferably in amounts mentioned above. Such a liposuction solution is traditionally called tumescent solution, although any buffered aqueous solution used in liposuctions is sometimes called as "tumescent solution", regardless of whether lidocaine or adrenaline/epinephrine is present or not. Therefore, the terms "liposuction solution" and "tumescent solution" as used herein are interchangeable, unless otherwise indicated. It follows that in some further embodiments, a liposuction liquid containing neither lidocaine (or any other local anesthetic) nor adrenaline/epinephrine may still be called as "tumescent solution".

The term "spent liposuction solution" refers to a liposuction solution that has been used in liposuction, i.e. that has been in contact with human subcutaneous fat *in vivo* and/or after aspiration *ex vivo.* The spent liposuction solution comprises various at least partly water-soluble substances originating from the subcutaneous fat tissue.

When a lipoaspirate obtained by a liposuction technique that involves use of a liposuction solution is centrifuged or allowed to stand for a while, the lipoaspirate separates into different layers, namely into a lipid layer on the top, an adipose tissue layer comprising adipocytes and other cells of the adipose tissue in the middle, and an aqueous layer at the bottom. The aqueous layer contains the spent liposuction solution used for the liposuction. Any cell or tissue debris contained in the lipoaspirate may separate into a fourth layer below the aqueous layer. In this context, the term "layer" can be used interchangeably with the term "fraction".

The cell-free preparation of the invention is obtainable by a method in which a lipoaspirate is processed in a manner that results in a preparation that contains a lipid fraction and/or an aqueous fraction of the lipoaspirate, and is devoid of cells naturally present in an adipose tissue. This may be achieved in different ways. In some embodiments, the method comprises the steps of removing the adipose tissue fraction of a lipoaspirate, collecting the remaining lipid fraction and/or aqueous fraction, and sterile filtering the collected fraction(s). In some other embodiments, the adipose tissue fraction is not actively removed but only the desired fractions, i.e., either the lipid fraction, the aqueous fraction, both are collected and subsequently sterile-filtered. Since no washing steps are carried out prior to collecting the aqueous fraction, it contains the spent liposuction solution. It follows, that the collected fraction may or may not contain a local anesthetic, such as lidocaine, and/or adrenaline. It also follows that the lipid fraction is not removed by any washing steps. In some embodiments, the lipoaspirate is shaken gently and/or allowed to settle such that it separates into different layers prior to removing the adipose tissue fraction and/or collecting the lipid/or the aqueous fraction. The adipose tissue fraction can be removed by any suitable technique available in the art. Also collecting of the lipid fraction and/or the aqueous fraction can be carried out by any suitable technique available in the art, Sterile filtering is usually carried out by using a 0.22 µm filter.

In some embodiments, liposuction solution used in the liposuction but not aspirated together with the adipose tissue (i.e., is collected to a separate container during or right after the liposuction) may be provided as the present cell-free preparation or used as an aqueous component thereof, for example by way of being added to the collected aqueous fraction of a lipoaspirate to increase its volume. In some further embodiments, any physiologically acceptable aqueous buffered solution/saline may be added to the lipoaspirate to increase the volume of the aqueous fraction, if desired.

In some embodiments, the spent liposuction solution, either as a collected aqueous fraction of a lipoaspirate or as a spent liposuction solution not forming part of a lipoaspirate may be diluted or concentrated as desired by adjusting its volume using physiologically acceptable diluents and/or techniques readily available in the art. In some embodiments, the spent liposuction solution may be freeze-dried and reconstituted on demand.

In some embodiments, the preparation of the invention consists of a lipid fraction of a lipoaspirate.

In some embodiments, the preparation of the invention consists of an aqueous fraction of a lipoaspirate and/or of a spent liposuction solution not being part of a lipoaspirate.

In some embodiments, the of the invention consists of a lipid fraction and an aqueous fraction of a lipoaspirate with or without being supplemented with a spent liposuction solution not being part of the lipoaspirate.

Being cell-free means that the present preparation is fundamentally different from any adipose tissue-derived stromal vascular fraction (SVF) which contains heterogeneous cell populations such as mesenchymal progenitor/stem cells, preadipocytes, endothelial cells, pericytes, T cells, and M2 macrophages. The SVF is obtainable from the adipose tissue fraction of a lipoaspirate. Accordingly, the present preparation is also fundamentally different from nanofat, which is an emulsified and filtered adipose tissue fraction of a lipoaspirate, as well as from any cell-free extracts obtained from nanofat by removal of the cells.

Moreover, the present cell-free preparation is fundamentally different from any decellularized fat-derived compositions available in the art. Such decellularized compositions are natural scaffolds derived from adipose tissue, in which the cellular and nuclear contents are eliminated, but the three-dimensional structure and composition of the extracellular matrix (ECM) are preserved.

The preparation of the invention has several advantages by way of being cell-free, including for example, low immunogenicity and hence reduced risk of adverse effects when administered to a human body. It follows that the preparation of the invention may be provided as an allogenic product, i.e., as a product suitable for use by subjects different from the donor of the lipoaspirate. There are also many practical benefits when factors such as cell viability during storage or after administration need not be considered.

Earlier studies have indicated that an adipose tissue-derived cell-free extract can be used for soft tissue engineering and repair owing to its ability to induce both angiogenesis, i.e., formation of new blood vessels, and adipogenesis, i.e., formation of new adipose tissue.

It has now been unexpectedly realized that the cell-free lipoaspirate-derived preparation of the present invention is capable of promoting skin regeneration, a process that does not involve adipogenesis and that is affected not only by angiogenesis but is largely dependent also on other cellular processes such renewal of epidermal and dermal cells.

Accordingly, in some embodiments, the present cell-free preparation comprising or consisting of a lipid fraction and/or an aqueous fraction of a lipoaspirate may be denoted as a cell-free allogenic skin regeneration substitute.

Skin is the largest organ of the human body, consisting of three layers. The outermost layer of skin is epidermis, a thin protective layer consisting primarily of keratinocytes. The middle layer of skin is dermis, which makes up 90% of skin's thickness. The dermis is rich in collagen, a protein that makes skin cells strong and resilient, and in elastin, a protein that keeps the skin flexible and helps stretched skin regain its shape. The basement membrane zone between the epidermis and the dermis connects, and functionally separates, the epidermis and the dermis, and is indispensable for normal skin functions. The bottom layer of skin is hypodermis (i.e., subcutis), a fatty layer that cushions underlying muscles and bones. Hypodermal fat is arranged in the form of lobules separated from each other by fibrous septae that consist of blood vessels, nerves, lymphatics and connective tissue. Each lobule contains adipocytes (i.e., fat cells), which consist mostly of triglycerides.

As used herein, the term "hypodermal" refers to a matter derivable from human hypodermal tissue, i.e. subcutaneous tissue. The term may be used interchangeably with the terms "hypodermis-derived" or "subcutis-derived".

Since the preparation of the invention is derived from a lipoaspirate, it may also be denoted, for example, as a cell-free hypodermal preparation.

As used herein, the term "skin regeneration" refers to a natural process that occurs as the skin cells turn over. In other words, dead skin cells on the top layer of the epidermis fall away, revealing fresh, newly created cells beneath. Scar tissue forms when skin heals but it doesn't regenerate. As aging slows down the skin regeneration and changes the basement membrane zone, the skin becomes less elastic, thinner and more wrinkled or textured.

Without being limited to any theory, the preparation of the invention may promote skin regeneration, thereby improving management of scars and other skin defects, as well as diminishing visible signs of skin aging, by a number of different mechanisms of action. For example, the preparation not only brings essential structural components (i.e., building blocks) and nutrients to the skin but also induces the local cells to produce more essential factors. More specifically, it is envisaged that the preparation activates fibroblasts, cells that produce collagen, hyaluronic acid, and elastin, as well as induces local cell migration and differentiation. It also envisaged that the preparation activates keratinocytes and increases their turnover, thereby improving the barrier function of the skin. Moreover, the preparation is envisaged to improve functioning of the dermal-epidermal junction and repair thereof.

Aging skin becomes drier and thinner, loses its firmness and appears more wrinkled. The moisture content decreases in the outermost layer of the skin, which is partly due to the reduction of lipids, i.e. fatty substances, and partly due to decrease in hyaluronic acid. Both of these changes affect the skin such that it is no longer able to bind and retain water in the same way as a younger skin does. The cell-free preparation of the invention contains a wide number of different lipids, fatty acids, associated proteins, and proteins related to lipid metabolism, which are considered to help the skin retain moisture thereby reducing its dryness, thereby not only rejuvenating aged skin but also providing means to manage clinical and other skin conditions and defects that benefit from reduced dryness of the skin.

To be more specific, based on untargeted compositional analysis of the preparation, it essentially contains lipids, associated proteins and proteins related to lipid metabolism such as but not limited to ceramids, apolipoproteins, perilipins, non-saturated and saturated fatty acids, glycerophospholipids, lysophosphatidic acids, lysophospholipids, monoglycerides, diglycerides, triglycerides and prostaglandins. Some of these molecules may originate from the lipid fraction of a lipoaspirate, while some other molecules may originate from the aqueous fraction of a lipoaspirate. Accordingly, some of the components in the preparate may be fat-soluble while some other components may be water-soluble or partly water-soluble. Ceramics are particularly interesting components of the preparate according to the invention because they are lipids found in normal skin cells.

In accordance with the other proposed mechanisms of action, characterization of the preparation of the invention also revealed that it essentially contains also structural and non-structural extracellular matrix proteins, peptides, chains, subchains and/or subunits thereof, as well as proteins related to the synthesis of extracellular matrix. These essentially include but are not limited to Collagens I, III, IV, VI, XV, XVIII, fibronectin, vitronectin, elastin, hyaluronan, decorin, tenascin, laminin, lumican and prolargin. Interestingly, lumican is a a leucine-rich repeat proteoglycan that induces fibrillogenesis of collagen, whereas prolargin is a protein that anchors the basement membrane to the underlying tissue.

Interestingly, the preparate of the invention contains basement membrane proteins peptides, chains, subchains and/or subunits thereof, as well as specialized dermo-epidermal junction components, and factors effecting epidermis, including for example, without limitation, keratin, nidogen, versican, intergrins, periplakin, and plectin.

Notably, the preparate of the invention also contains essential and non-essential amino acids, antioxidants, vitamin derivatives and metabolites, and wide number of glycoproteins and proteoglycans as well as factors related to cell proliferation. Also these findings are in accordance with the proposed mechanisms of action, although the present invention is not limited to any theory or mechanism of action. Tretinoin is a particularly interesting component of the present preparate because it is an antioxidant that is famous for its anti-aging benefits and therefore often added into various skin care products. Tretinoin may also be classified into vitamins, more specifically vitamin derivatives or metabolites.

Surprisingly, neither VEGF, IGF-1 nor FGF-2 was among over 1004 proteins, over 6000 related peptides and over 500 metabolites identified as components of the present preparation on the basis of untargeted analysis by liquid chromatography-mass spectrometry (LC-MS).

The cell-free preparation of the invention may be applied for various therapeutic and/or cosmetic treatments of the skin. It may be used as such or as formulated into a pharmaceutical or a cosmetic composition.

As used herein, the term "pharmaceutical composition" refers broadly to a composition comprising the present cell-free lipoaspirate-derived preparation as a therapeutically active ingredient and one or more pharmaceutically acceptable components such as carriers, adjuvants and/or excipients. As used herein, the term "pharmaceutically acceptable" refers to a material that is suitable for administration to a human subject without undue adverse side effects such as toxicity, significant irritation and/or allergic responses. In other words, the benefit/risk ratio must be reasonable. In essence, the term "pharmaceutically acceptable" is interchangeable with the term "physiologically acceptable". Moreover, the pharmaceutically acceptable component should be such that it does not diminish the therapeutic activity of active ingredient, i.e., the present lipoaspirate-derived preparation.

As used herein, the term "cosmetic composition" refers broadly to a composition comprising the present cell-free lipoaspirate-derived preparation as an active ingredient and one or more cosmetically acceptable components such as carriers, adjuvants and/or excipients. As used herein, the term "cosmetically acceptable" refers to a material that is suitable for administration to a human subject without undue adverse side effects such as toxicity, significant irritation and/or allergic responses. In other words, the benefit/risk ratio must be reasonable. As readily understood by those skilled in the art, in order to be "cosmetically acceptable" the composition must also be "physiologically acceptable" as well as "dermatologically acceptable". Therefore, the terms can be used interchangeably.

Those skilled in the art to which the present invention belongs, can readily select appropriate pharmaceutically and/or cosmetically acceptable components available in the art, depending on the intended route of administration and formulation of the composition. The formulation may be carried out as desired using means and methods readily available in the art, for example by means of conventional mixing, dissolving, granulating, levigating, emulsifying, encapsulating, entrapping, lyophilizing or the like.

Excipients are preferably inert substances added to pharmaceutical and cosmetic compositions. Typical examples of different types of excipients, without limitation, include stabilizers, preservatives, pH modifiers, fillers, thickeners, viscosity modifiers, lubricants, solubilizers, surfactants, and the like.

Pharmaceutical and cosmetic compositions for topical administration include, but are not limited to, ointments, lotions, creams, gels, hydrogels, oil-in-water emulsions, water-in-oil emulsions, drops, sprays, liquids, solutions, powders and slow release or sustained release formulations, face masks, skin patches, mousses and foams.

Pharmaceutical and cosmetic compositions for parenteral administration are typically sterile aqueous or non-aqueous solutions, suspensions or emulsions to be applied topically or by injection, for example subcutaneously, intrahypodermally or intradermally. In some embodiments, the composition may be administered into different layers of the skin during the same treatment session and/or process. For example, the composition may be administered both by a subcutaneous, intrahypodermal and/or intradermal injection and by topical administration.

The composition, be it a pharmaceutical or a cosmetic composition, may also be provided in a concentrated form or in a form of a powder (i.e. lyophilized) to be reconstituted on demand, regardless of whether the composition is intended for topical or parenteral administration.

The present invention provides the cell-free lipoaspirate-derived preparation disclosed herein for use in aesthetic dermatology. This aspect of the invention may be expressed, for example, as a use of said cell-free preparation or a cosmetic composition comprising the same for cosmetic treatment of the skin or as a non-therapeutic method for cosmetically treating the skin. The method comprises a step of applying said preparation or said composition to the skin to be treated, preferably in a cosmetically efficient amount. In some embodiments, the skin to be treated is compromised skin.

As used herein, the expressions "cosmetic treatment" and "non-therapeutic treatment" may be used interchangeably, both referring to the administration of the present cell-free preparation or a cosmetic composition comprising the same to a subject in need thereof for a purpose which may include promoting skin regeneration and/or skin rejuvenation. Improved skin regeneration and/or rejuvenation may manifest itself, without limitation, as reduced dark circles around the eyes, reduced dryness and roughness of the skin, reduced wrinkles and fine lines, decreased redness of the skin, reduced number, size and/or darkness of age spots (liver spots) and other hyperpigmentation defects, reduced number, size and/or whiteness of vitiligo patches, decreased cellulite, burns, improved stretch marks and scars, such as scars associated with acne or other clinical skin conditions, or scars caused by injuries or operations.

As used herein, the term "cosmetically efficient amount" refers to an amount by which signs of cosmetic defects, such dark areas around the eyes, dryness and roughness of the skin, wrinkles and fine lines, redness of the skin, age spots (liver spots) and other hyperpigmentation defects, vitiligo patches, cellulite, stretch marks and scars, are at least reduced or ameliorated.

As used herein, the term "compromised skin" refers to the skin whose outer layer, i.e., the epidermis, has been damaged for any reason. In relation to aesthetic dermatology, the skin to be treated may be comprised owing a cosmetic treatment such as microneedling, laser treatment or exfoliation, or owing to aging.

In a further aspect, the present invention provides the cell-free preparation disclosed herein for use in clinical dermatology. This aspect of the invention may be expressed, for example, as a cell-free preparation or a pharmaceutical composition comprising the same for use in treating a clinical skin disorder or condition, or as a therapeutic method for treating a clinical skin disorder or condition, typically exhibiting compromised skin. The method comprises a step of applying the preparation or the composition to the skin to be treated, preferably in a therapeutically efficient amount.

As used herein, the expression "therapeutic treatment" in its different linguistic forms refers to the administration of the present cell-free preparation or a pharmaceutical composition comprising the same to a subject in need thereof for a purpose which may include ameliorating, lessening, inhibiting, or curing a clinical skin disorder or condition.

As used herein, the term "clinical skin condition" refers to a clinical dermatologic disorder, condition or defect that typically manifests itself as compromised skin, and often exhibits an undesirable, unsightly skin appearance. Such skin conditions often involve inflammation, such as that caused by a bacterial, fungal or viral infection. Clinical skin conditions that are envisaged to benefit from improved skin regeneration by the present cell-free preparation or a pharmaceutical or cosmetic composition comprising the same include, but are not limited to Acne, Actinic Keratosis, Atopic Dermatitis, venous stasis dermatitis, eczema, Contact Dermatitis, Melasma, Vitiligo, Psoriasis, Rosacea, Seborrheic Dermatitis, and Epidermolysis bullosa.

As used herein, the term "therapeutically efficient amount" refers to an amount by which harmful effects of the skin condition are, at a minimum, ameliorated.

Those skilled in the art will readily understand that cosmetic and therapeutic treatments may often overlap. Moreover, some skin conditions may be considered as both clinical dermatological conditions or defects and aesthetic skin conditions and defects. Both types of skin conditions and defects, regardless of how they are classified, benefit from the treatment with the preparation of the invention.

In some embodiments of the above-mentioned therapeutic and/or cosmetic uses and methods, the present preparation or a composition comprising the same is to be applied topically onto the skin to be treated, such as onto compromised skin. In some embodiments, said methods may comprise the steps of i) preparing compromised skin, for example by a treatment that involves microneedling, exfoliation or laser resurfacing, and ii) applying the preparation or the composition on the compromised skin so prepared. In some other embodiments, said methods may comprise a step of applying the preparation or the composition on pre-compromised skin, i.e. on skin that has been prepared earlier, for example by any of the treatments mentioned above, said compromising treatments thus not forming part of the therapeutic and/or cosmetic treatment of some embodiments of the invention.

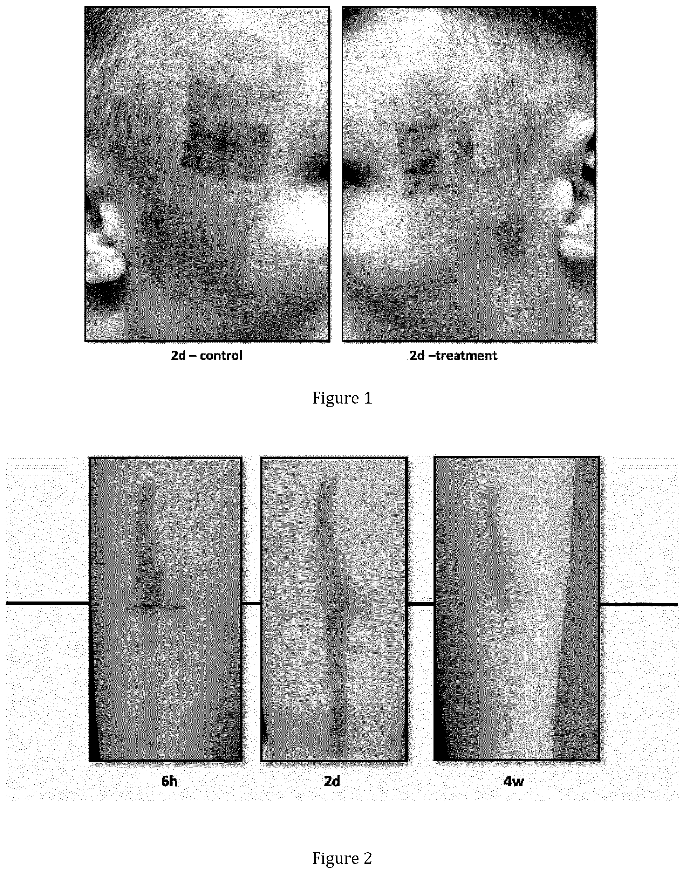

In accordance with what is stated above, when the preparation of the invention is applied to skin areas associated with acne or other scars, preferably compromised e.g. by laser resurfacing, the healing process becomes faster, the scars fade and a healthy skin appearance is observed. Indeed, faster healing process in response to administration of the preparation of the invention onto laser resurfaced facial areas associated with acne scars is demonstrated in Figure 1. Moreover, fading of scars and improved skin appearance is demonstrated in Figure 2. To conclude, these results show that preparation of the invention has the potential to promote skin regeneration.

Furthermore, when the preparation of the invention is applied to skin areas, preferably compromised e.g. by microneedling, reduction of wrinkles, pores, UV spots, brown spots and red areas is observed. These results show that preparation of the invention has the potential to promote skin regeneration and/or skin rejuvenation.

In some further embodiments of the above-mentioned therapeutic and/or cosmetic uses and methods, the present preparation or a composition comprising the same is to be applied intradermally into the skin to be treated, such as aged skin or photodamaged skin, for example as mesotherapy. Also in such embodiments, improvement in skin quality, health and appearance is observed as judged, without limitation, by reduction of wrinkles, pores, UV spots, brown spots and red areas. Also these results demonstrate that preparation of the invention has the potential to promote skin regeneration and/or skin rejuvenation.

In one aspect, the present invention provides a method of manufacturing a cosmetic composition comprising the cell-free preparate disclosed herein for a non-therapeutic treatment of skin, such as compromised or non-compromised skin including aged skin.

### EXAMPLE 1. Preparation and characterization a cell-free hypodermal preparation

Human lipoaspirate samples were obtained with a signed informed consent from three healthy volunteers undergoing standard water-assisted liposuction.

The lipoaspirates were processed without rinsing by first subjecting them to gentle shaking, and then by allowing the shaken lipoaspirates to separate into a lipid fraction on the top, an adipose tissue fraction containing adipocytes and other cells in the middle, and an aqueous fraction containing the used liposuction solution at the bottom. The middle fraction was removed, whereas the lipid fraction and the aqueous fraction were collected and combined, followed by sterile filtering using a 0.22 µm filter. The cell-free preparations so obtained were stored at -20°C in aliquots.

Three independent patches from three different donors so obtained were subjected to untargeted metabolomics analysis, a comprehensive analysis of all the measurable analytes in a sample including chemical unknowns, by using UPLC-MS platform.

Sample preparation was carried out as follows: samples of the three batches were thawed on ice, followed by transferring 100 µL of each sample into a new tube and addition of 300 µL methanol. Next, the samples were vortexed for 30 s, treated by sonication for 30 min at 4°C, and kept at -20°C for 1 hour. Next, the samples were vortexed for 30 s, and kept at -20 °C for 0.5 h. Thereafter, the samples were centrifuged at 12000 rpm for 15 min at 4 °C. Finally, 200 µL of supernatant and 5 µL of DL-o-Chlorophenylalanine (0.5 mg/mL) was transferred to avial for LC-MS analysis.

LC-MS Analysis was carried out as follows: separation was performed by Waters Acquity UPLC combined with Q Exactive MS (Thermo) and screened with ESI-MS. The LC system was comprised of ACQUITY UPLC HSS T3 (100 × 2.1 mm × 1.8 µm) with Acquity UPLC. The mobile phase was composed of solvent A (0.05% formic acid water) and solvent B (acetonitrile) with a gradient elution (0-1.0 min, 5%B; 1.0-12.5 min, 5%-95%B; 12.5-13.5 min, 95%B; 13.5-13.6 min, 95%-5%B; 13.6-16.0 min, 5%B). The flow rate of the mobile phase was 0.3 mL/min. The column temperature was maintained at 40 °C, and the sample manager temperature was set at 4 °C.

Mass spectrometry parameters in ESI+ and ESI- mode were following:
ESI+: Heater Temp 300 °C; Sheath Gas Flow rate, 45arb; Aux Gas Flow Rate, 15arb; Sweep Gas Flow Rate,1arb; spray voltage, 3.0KV; Capillary Temp, 350 °C; S-Lens RF Level, 30%.
ESI-: Heater Temp 300 °C, Sheath Gas Flow rate, 45arb; Aux Gas Flow Rate, 15arb; Sweep Gas Flow Rate, 1arb; spray voltage, 3.2KV; Capillary Temp,350 °C; S-Lens RF evel, 60%.

Based on the analysis, the samples contained over 1004 proteins and over 6000 related peptides and over 500 metabolites. Surprisingly, neither VEGF, IGF-1 nor FGF-2 was among the identified proteins or peptides.

Instead, the samples contained various lipids, associated proteins and proteins related to lipid metabolism; various basement membrane components and proteins and peptides related to dermo-epidermal junction or proteins and peptides affecting dermis and epidermis; essential and non-essential amino acids, antioxidants, vitamins, and larger number of glycoproteins and proteoglycans as well as factors related to cell proliferation. Some of the compounds identified are regarded as endogenous, some exogenous. However, all the component originate from the donor tissue. It also to be noted that some of the components are intracellular, some extracellular.

Some of the identified molecules are listed in Table 1 below.

**Table 1.**

| | |
|---|---|
| **Structural and non-structural extracellular matrix proteins and proteins related to the synthesis of ECM** | Collagens I, IV, VI, XV, XVIII (e.g. Collagen alpha-1(I) chain, Collagen alpha-3(VI) chain, Collagen alpha-1(XVIII) chain, Collagen alpha-1(XV) chain), Fibronectins, vitronectin, decorin, laminins (e.g. Laminin subunits beta-1, Laminin subunit beta-2, Laminin subunit gam Laminin subunit alpha-4), Connective tissue-activating peptide III, Biglycan, Tenascin-X, Fibulin-1, Lumican, Prolargin, EGF-containing fibulin-like extracellular matrix protein 1, Periostin, Transforming growth factor-beta-induced protein ig-h3, Asporin |
| **Lipids or associated proteins** | Ceramides (e.g. Cer 18:0;2O/14:0, Cer 8:1;2O/26:3, CerP 18:1;2O/2:0, CerP 19:0;2O/2:0), Apolipoproteins (e.g. Apolipoprotein L1, Apolipoprotein M, Apolipoprotein A-I, Apolipoprotein E, Apolipoprotein A-II, Apolipoprotein C-I, Apolipoprotein C-II, Apolipoprotein C-III, Apolipoprotein B-100;Apolipoprotein B-48, Apolipoprotein D, Apolipoprotein A-IV, Apolipoprotein F) , Adipogenesis regulatory factor, Fatty acid-binding protein, Fatty acid synthase, Galectin-3, Long-chain-fatty-acid--CoA ligase 1, Phospholipid transfer protein, Serine palmitoyltransferase 3, Serine palmitoyltransferase 2 , Perilipin-1, Perilipin-3, Perilipin-4, clusterin |
| | |
| | Fatty acids (medium and long chains, saturated and unsaturated): (e.g. 5-phenylvaleric acid, Adipate, Maleamic acid, Palmitic Acid, Palmitoleic Acid, Tetradecanedioic acid, Pentadecanoic acid, Tridecanoic acid, Tridecylic acid, Undecanoic acid, Undecanedioic acid) |
| | |
| | Monoglycerophospholipids (e.g. LPC 10:0, LPC 10:0-SN1, LPC 11:0, LPC 11:0-SN1, LPC 12:0, LPC 12:0-SN1, LPC 13:0, LPC 14:0, LPC 14:0-SN1, LPC 16:0, LPC 16:1, LPC 16:1-SN1, LPC 16:4-SN2, LPC 18:0, LPC 18:1, LPC 18:2, LPC 20:4, LPC 20:4-SN1, LPC O-16:1, LPC O-16:2, LPC 0-18.1) |
| | |
| | Prostaglandins: Prostaglandin F2, Prostaglandin F2α 1-11-lactone |
| | Prostaglandin F3, Prostaglandin E synthase 3 |
| | Sebacic acid? |
| | Lysophosphatidic acids (e.g. LPA 12:0, LPA 13:0) |
| | Lysophospholipids (e.g. LPE 14:0, LPE 18:0, LPE 18:1, LPE 18:2, LPE 20:4) |
| | |
| | Lipid metabolism related proteins (e.g. Glycerol-3-phosphate dehydrogenase 1-like protein, Glycerol-3-phosphate dehydrogenase) |
| | |
| | Glycerophospholipids (e.g. PC 12:0_12:0, PC 18:0_18:1, PC 18:1_18:1, PC 34:1; PC 16:0-18:1, PC O-12:0, PC O-36:2, PC(16:0/0:0), PA 22:3; PA 6:0-16:3) |
| | |
| | Diacylglycerols (e.g. DG 28:2, DGDG 30:5; DGDG 12:0-18:5, DGDG 0-9:0_22:4) |
| **Basement membrane/ Dermo-epidermal junction components, factors effecting skin/epidermis** | Keratin type II, ceramides, Versican, Periplakin, Collagen alpha-1(XV) chain, Integrin-linked protein kinase, periplakin, Nidogen-1, Nidogen-2, Integrin beta-8, Prolargin, Basement membrane-specific heparan sulfate proteoglycan core protein, Plectin, SUMO-activating enzyme subunit 2, Talin-1, Talin-2 |
| **Amino acids** | Non-essential amino acids (e.g. Asparagine, Cysteine, Isodityrosine, Methionine, Taurine, Tyrosine, Pipecolic acid Essential amino acids (e.g. Threonine, Isoleucine, Leucine, Tryptophan, Norleucine, Norvaline) |
| **Vitamins and peptides with antioxidant properties or related molecules/ metabolites** | Vitamin A related: Tretinoin, Retinal dehydrogenase 1, Retinol-binding protein 4, Transthyretin; Vitamin D related: Vitamin D - binding protein; Vitamin B related: Nicotinamide (Vitamin B3), Biotinidase, Pyridoxamine, Pantothenic acid; Selenoprotein P, Pyroglutamic acid |

### Example 2. Safety study

The purpose of the safety study was to evaluate the tolerability of the present allogenic lipoaspirate-derived preparation and to reveal possible mid-term adverse events (AEs) related to its use.

Three independent batches disclosed in Example 1 were used in the study to ensure the uniformity of the preparations also in the safety perspective.

The study was conducted as a randomized, double-blinded, placebo-controlled study at a private sector hospital, Helsinki, Finland, with signed informed consent form the study subjects.

To this end, a total of 38 volunteers (74% women) were recruited. The subjects represented all five skin types according to the Fitzpatrick classification, with an emphasis on categories two and three. 34% of the study subjects had some underlying disease such as hypertension, migraine or asthma, the rest were considered totally healthy. No one had an underlying disease that could affect the interpretation of the results. Subjects who had previously been diagnosed with an allergic reaction leading to anaphylaxis, as well as those with acute cancer were excluded from the study. Moreover, the subjects were not allowed to use any medication to treat allergies or resistance during the study to guarantee the reliability of the results.

Subcutaneous injections were chosen as the route of administration as that administration route is generally associated with a higher risk of AEs as compared to topical administration. A high dose (3-10 times the amount intended for normal use) of the test preparation was injected into the subcutaneous tissue of each subject's arm. The other side of the arm was injected with a corresponding amount of Ringer-Acetat solution, which served as a placebo. Ringer Acetat has not been associated with AEs and is generally considered as safe and routinely used for intravenous administration.

The study subjects were monitored for three months using an electronic diary, calls by research nurse and doctor's visits. The follow-up was initially daily and then monthly. The study was carried out in cooperation with a clinical research unit of a private sector hospital and was monitored by a independent contract research organization (CRO).

Both allergic reactions and irritation symptoms were extensively investigated in the study. All adverse reactions were reported, evaluated by the physician, and analyzed. The subjects measured their body temperature every day for a week, and none reported fever that was related to the administration of the test preparation. Also, no injection site infections were observed during the study. Immediately after the injection, the test preparation was found to cause slightly more redness at the injection site than the placebo.

Based on some earlier studies, the test preparation was known to cause temporary vasoconstriction, i.e., pale skin color in the injection area due to constriction of blood vessels. This effect is related to the preparations mode of action and is not considered an AE or does not predict later AEs. Vasoconstriction events were reported also during the safety study.

Swelling and bruising were observed more related to the placebo injections than to the test injection, indicating that these reactions were primarily related to the route of administration (injection) and fluid accumulation into the tissue. Reported acute AEs are summarized in Table 2 below,

**Table 2. Acute adverse reactions**

| **Day** | **Number of reported reactions** | **Test preparation** | **Placebo** |
|---|---|---|---|
| Day 0 (n = 38) | Hematoma | 7 | 11 |
| | Erythema | 3 | 1 |
| | Edema | 2 | 2 |
| | Tingling | 1 | 0 |
| | Abnormal injection mark | 1 | 0 |
| | Injection site pain | 4 | 2 |
| Day 1 (n = 37) | Hematoma | 12 | 19 |
| | Erythema | 1 | 0 |
| | Edema | 1 | 1 |
| | Injection site pain | 2 | 4 |
| Day 2 (n = 37) | Hematoma | 11 | 18 |
| | Erythema | 1 | 0 |
| | Edema | 0 | 1 |
| | Injection site pain | 0 | 1 |
| Day 3 (n = 38) | Hematoma | 10 | 20 |
| | Erythema | 1 | 1 |
| | Edema | 0 | 1 |
| Day 4 (n = 38) | Hematoma | 9 | 18 |
| | Erythema | 1 | 1 |
| | Edema | 0 | 1 |
| Day 5 (n = 38) | Hematoma | 8 | 18 |
| | Erythema | 0 | 1 |
| | Abnormal injection mark | 0 | 1 |
| Day 6 (n = 37) | Hematoma | 6 | 15 |
| | Erythema | 1 | 1 |
| Day 7 (n = 38) | Hematoma | 7 | 15 |
| | Erythema | 1 | 0 |

In the three-month follow-up, no surprising or serious AEs were observed and nothing abnormal was observed in the doctor's assessment at day 90. The product was found to cause no mid-term AEs at all, particularly evaluated in terms of pigmentation, induration, necrosis, granuloma, or other abnormalities. No significant differences were found between the three batches used in terms of AEs, confirming that the preparation of the invention has a uniform safety profile with no batch-to-batch variation.

### Example 3. Improved overall skin healing and regeneration after laser

Cell-free lipoaspirate-derived preparations disclosed in Example 1 were tested for their ability to improve overall healing of the skin and improve scar appearance after laser resurfacing with eight volunteers (aged 22-72 years) who were treated essentially in the same manner as the first subject.

Figure 1 shows photographs of right and left profiles of a male (age 22) whose temple and cheek areas were treated with fractional CO2-laser to reduce the appearance of acne scars. Both sides of the face were treated with the same settings. After the treatment, the present cell-free lipoaspirate-derived preparation (0.5 ml) was topically applied to the left side of the face (the photograph on the right), whereas the right side of the face was treated with sterile water (the photograph on the left). The photographs illustrate the healing and re-epithelization process 2 days after the treatment. Improved recovery and faster healing on the area treated with the preparation of the invention was observed.

Figure 2 shows photographs of a four times operated leg. A three year old scar was treated with fractional CO2 laser using the same settings for the whole scar. The lower half of the scar was treated twice by topical administration of the present cell-free lipoaspirate-derived preparation (0.5ml), first immediately after the laser treatment and then 6 hours later for the second time. At 6h time point, vasoconstriction was seen in the scar area treated with the preparation of the invention. 2 Days after the laser resurfacing, the scar area treated with the preparation of the invention was less red and rough as compared to the non-treated area. 28 Days after the laser resurfacing, the area scar area treated with the preparation of the invention was much was lighter in color, softer and more at the skin level as compared to the non-treated scar area.

The other six subjects also reported faster recovery and less pain in the treated areas.

### Example 4. Promotion of skin regeneration in microneedled skin

Cell-free lipoaspirate-derived preparations disclosed in Example 1 were tested on three healthy volunteers with signed informed consent, who underwent standard facial microneedling, a widely used cosmetic procedure that is used to encourage collagen and elastin production using small, sterilized needles, for improving the appearance and texture of the skin. The follow up time was 4 weeks.

The first subject was a 34 year old female with 13 years old facial scars that had been treated earlier with microneedling without any improvement. Now the whole face, including the scar area was treated with microneedling (Dermapen 0.7mm -2mm depth) and right after that the preparation of the invention was applied topically onto the left side of the face (ca. 1 ml). VISIA pictures were taken prior to the treatment using standard angle and light.

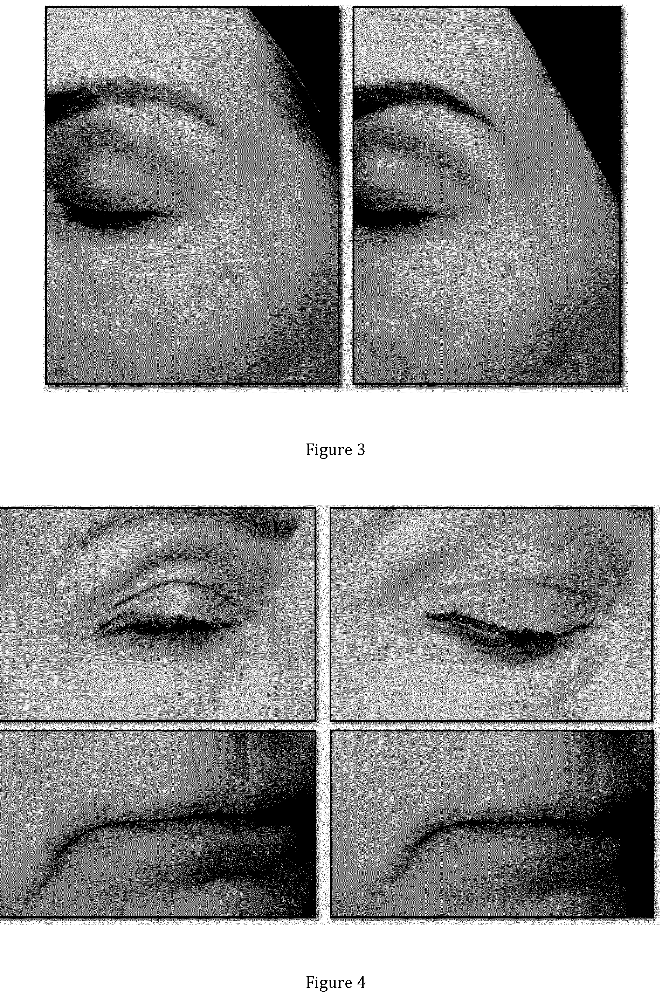

At a control visit 4 weeks after the treatment, the first subject reported significant improvement in the appearance of the scar area. Before and after photographs are shown in Figure 3. At the same visit, new VISIA pictures were taken again using standard angle and light, and compared to the previous pictures. According to the VISIA analysis, patient showed improvement in skin texture, pores and red areas up to 31%.

The other two subjects (aged 59 and 72) were treated essentially in the same manner as the first subject. In VISIA analysis, they showed improvement in texture, pores and red areas up to 25%.

All subjects reported excellent outcomes in subjective evaluation.

### Example 5. Mesothermal treatments of aged skin

Cell-free lipoaspirate-derived preparations prepared as described in Example 1 were tested for their skin regeneration and/or rejuvenation potential on three healthy volunteers with signed informed consent. The subjects had non-compromised but aged skin. Each subject was treated with a different batch of the three batches prepared.

The first subject was a 58-old female who was treated with mesotherapy (sometimes called intradermatherapy or multi-puncture treatment). Circa 70 papulas (volume 0.04ml/papula) were injected to the whole face area. Four weeks after the treatment, significant improvement in skin quality (soft, "velvet-kind of" texture) was observed subjectively as well as in a VISIA picture (standarsd angle and light). In addition, vanishing of vertical lines around the mouth area as well as in the eye area was observed. According to the VISIA analysis, texture and spots were improved up to 23%.

In overall VISIA analysis, skin age was decreased for two years. Before and after photographs are shown in Figure 4.

The two other subjects (aged 67 and 70) were treated essentially in the same manner as the first subject. In VISIA analysis, texture and UV spots were improved up to 7% and skin age decreased up to two years.

All subjects reported excellent outcomes in subjective evaluation.

The second treatment was performed 6 weeks after the first one. Three weeks after the second treatment, the subjects were still very pleased and reported excellent outcomes in subjective evaluation.

## Claims

1. A cell-free lipoaspirate-derived preparation **characterized in that** the preparation is sterile-filtered and comprises at least one of a lipid fraction and an aqueous fraction of a lipoaspirate.

2. The cell-free preparation according to claim 1, wherein the aqueous fraction comprises spent liposuction solution, with or without a local anesthetic and/or adrenaline.

3. The cell-free preparation according to claim 1 or 2, wherein the preparation comprises one or more components selected from the group consisting of structural and non-structural extracellular matrix proteins and peptides, chains and subunits of said extracellular matrix proteins; ; lipids, lipid-associated proteins, and proteins related to lipid metabolism selected from the group consisting of ceramids, apolipoproteins, perilipins, non-saturated and saturated fatty acids, glycerophospholipids, lysophosphatidic acids, lysophospholipids, monoglycerides, diglycerides, triglycerides and prostaglandins; Basement membrane proteins and peptides chains, and subunits of said basement membrane proteins; specialized dermo-epidermal junction components; essential and non-essential amino acids; antioxidants; Tretinoin, Retinal dehydrogenase 1, Retinol-binding protein 4, Transthyretin, Vitamin D -binding protein, Nicotinamide (Vitamin B3), Biotinidase, Pyridoxamine, Pantothenic acid, Selenoprotein P, and Pyroglutamic acid; and glycoproteins and proteoglycans.

4. The cell-free preparation according to claim 3, wherein
i) said structural and non-structural extracellular matrix proteins and peptides, chains, and subunits of said extracellular matrix proteins include one or more component selected from the group consisting of Collagens I, III, IV, VI, XV, XVIII, fibronectin, vitronectin, elastin, hyaluronan, decorin, tenascin, laminin, lumican and prolargin; and/or
ii) said basement membrane proteins and peptides, chains and subunits of said basement membrane proteins and said dermo-epidermal junction components include one or more component selected from the group consisting of keratin, nidogen, versican, intergrins, periplakin, and plectin.

5. The cell-free preparation according to any one of claims 1-4, wherein the preparation is obtainable by a method comprising the steps of:
- optionally, shaking a human lipoaspirate;
- removing an adipose tissue fraction of the lipoaspirate, said fraction comprising cells; and
- sterilizing remaining fractions of the lipoaspirate by filtration.

6. A cosmetic or pharmaceutical composition comprising the cell-free preparation according to any one of claims 1-5 and a physiologically acceptable carrier, adjuvant and/or excipient.

7. The cosmetic or pharmaceutical composition according to claim 6, wherein the composition is in the form of an ointment, lotion, cream, gel, hydrogel, oil-in-water emulsion, water-in-oil emulsion, drop, spray, liquid, solution, powder, slow release or sustained release formulation, face mask, skin patch, mousse or foam.

8. The cosmetic or pharmaceutical composition according to claim 6 or 7, wherein the composition is intended for topical administration, or for subcutaneous, intradermal or intrahypodermal administration.

9. Use of the cell-free preparation according to any one of claims 1-5 or the cosmetic composition according to any one of claims 6-8 for cosmetically treating the skin.

10. A non-therapeutic method for cosmetically treating the skin, the method comprising the step of applying the cell-free preparation according to any one of claims 1-5 or the cosmetic composition according to any one of claims 6-8 to the skin.

11. The use according to claim 9 or the method according to claim 10, wherein the skin is compromised skin, preferably prepared by microneedling, exfoliation and/or laser resurfacing.

12. The use according to claim 9 or the method according to claim 10, wherein the skin is aged skin.

13. The cell-free preparation according to any one of claims 1-5 or the pharmaceutical composition according to any one of claims 6-8 for use in treating a clinical skin condition.

14. The cell-free preparation or the pharmaceutical composition for use according to claim 13, wherein the clinical skin condition is Acne, Actinic Keratosis, Atopic Dermatitis, venous stasis dermatitis, Ezcema, Contact Dermatitis, Melasma, Vitiligo, Psoriasis, Rosacea, Seborrheic Dermatitis, or Epidermolysis bullosa.

15. A method of manufacturing a cosmetic composition for a non-therapeutic treatment of skin comprising the cell-free preparation according to any one of claims 1-5.

## Patentansprüche

1. Zellfreie lipoaspiratabgeleitete Zubereitung,
**dadurch gekennzeichnet, dass** die Zubereitung sterilfiltriert ist und mindestens eine einer Lipidfraktion und einer wässrigen Fraktion eines Lipoaspirats umfasst.

2. Zellfreie Zubereitung nach Anspruch 1, wobei die wässrige Fraktion verbrauchte Liposuktionslösung mit oder ohne Lokalanästhetikum und/oder Adrenalin umfasst.

3. Zellfreie Zubereitung nach Anspruch 1 oder 2, wobei die Zubereitung eine oder mehrere Komponenten umfasst, die ausgewählt sind aus der Gruppe bestehend aus strukturellen und nicht-strukturellen extrazellulären Matrixproteinen und -peptiden, Ketten und Untereinheiten der genannten extrazellulären Matrixproteine; ; Lipiden, Lipid-assoziierten Proteinen und Proteinen, die mit dem Lipidstoffwechsel zusammenhängen, die ausgewählt sind aus der Gruppe bestehend aus Ceramiden, Apolipoproteinen, Perilipinen, ungesättigten und gesättigten Fettsäuren, Glycerophospholipiden, Lysophosphatidsäuren, Lysophospholipiden, Monoglyceriden, Diglyceriden, Triglyceriden und Prostaglandinen; Basalmembranproteinen und -peptidketten und Untereinheiten der genannten Basalmembranproteine; spezialisierten dermoepidermalen Verbindungskomponenten; essentiellen und nicht-essentiellen Aminosäuren; Antioxidantien; Tretinoin, Retinaler Dehydrogenase 1, Retinol-bindendem Protein 4, Transthyretin, Vitamin-D-bindendes Protein, Nicotinamid (Vitamin B3), Biotinidase, Pyridoxamin, Pantothensäure, Selenoprotein P und Pyroglutaminsäure; und Glykoproteinen und Proteoglykanen.

4. Zellfreie Zubereitung nach Anspruch 3, wobei
i) die strukturellen und nicht-strukturellen extrazellulären Matrixproteine und -peptide, Ketten und Untereinheiten der extrazellulären Matrixproteine eine oder mehrere Komponenten einschließen, die ausgewählt sind aus der Gruppe bestehend aus den Kollagenen I, III, IV, VI, XV, XVIII, Fibronektin, Vitronektin, Elastin, Hyaluronan, Decorin, Tenascin, Laminin, Lumican und Prolargin; und/oder
ii) die Basalmembranproteine und -peptide, Ketten und Untereinheiten der Basalmembranproteine und die dermoepidermalen Verbindungskomponenten eine oder mehrere Komponenten einschließen, die ausgewählt sind aus der Gruppe bestehend aus Keratin, Nidogen, Versican, Intergrinen, Periplakin und Plectin.

5. Zellfreie Zubereitung nach einem der Ansprüche 1 bis 4, wobei die Zubereitung erhältlich ist durch ein Verfahren umfassend die Schritte:
- wahlweise Schütteln eines menschlichen Lipoaspirats;
- Entfernen einer Fettgewebefraktion des Lipoaspirats, wobei die Fraktion Zellen umfasst; und
- Sterilisieren der verbleibenden Fraktionen des Lipoaspirats durch Filtration.

6. Kosmetische oder pharmazeutische Zusammensetzung, umfassend die zellfreie Zubereitung nach einem der Ansprüche 1 bis 5 und einen physiologisch verträglichen Träger, Adjuvans und/oder Exzipienten.

7. Kosmetische oder pharmazeutische Zusammensetzung nach Anspruch 6, wobei die Zusammensetzung in Form einer Salbe, Lotion, Creme, eines Gels, Hydrogels, einer Öl-in-Wasser-Emulsion, einer Wasser-in-Öl-Emulsion, eines Tropfens, eines Sprays, einer Flüssigkeit, einer Lösung, eines Pulvers, einer Formulierung mit langsamer oder anhaltender Freisetzung, einer Gesichtsmaske, eines Hautpflasters, einer Mousse oder eines Schaums vorliegt.

8. Kosmetische oder pharmazeutische Zusammensetzung nach Anspruch 6 oder 7, wobei die Zusammensetzung zur topischen Verabreichung oder zur subkutanen, intradermalen oder intrahypodermalen Verabreichung vorgesehen ist.

9. Verwendung der zellfreien Zubereitung nach einem der Ansprüche 1 bis 5 oder der kosmetischen Zusammensetzung nach einem der Ansprüche 6 bis 8 zum kosmetischen Behandeln der Haut.

10. Nicht-therapeutisches Verfahren zum kosmetischen Behandeln der Haut, das Verfahren umfassend den Schritt des Auftragens der zellfreien Zubereitung nach einem der Ansprüche 1 bis 5 oder der kosmetischen Zusammensetzung nach einem der Ansprüche 6 bis 8 auf die Haut.

11. Verwendung nach Anspruch 9 oder das Verfahren nach Anspruch 10, wobei die Haut geschädigte Haut ist, die vorzugsweise durch Mikronadelung, Peeling und/oder Laser-Resurfacing vorbereitet wurde.

12. Verwendung nach Anspruch 9 oder das Verfahren nach Anspruch 10, wobei die Haut gealterte Haut ist.

13. Zellfreie Zubereitung nach einem der Ansprüche 1 bis 5 oder die kosmetische Zusammensetzung nach einem der Ansprüche 6 bis 8 zur Verwendung beim Behandeln eines klinischen Hautzustands .

14. Zellfreie Zubereitung oder die pharmazeutische Zusammensetzung zur Verwendung nach Anspruch 13, wobei der klinische Hautzustand Akne, aktinische Keratose, atopische Dermatitis, venöse Stauungsdermatitis, Ekzem, Kontaktdermatitis, Melasma, Vitiligo, Psoriasis, Rosacea, seborrhoische Dermatitis oder Epidermolysis bullosa ist.

15. Verfahren zum Herstellen einer kosmetischen Zusammensetzung zur nicht-therapeutischen Behandlung der Haut, umfassend die zellfreie Zubereitung nach einem der Ansprüche 1 bis 5.

## Revendications

1. Préparation acellulaire dérivée de lipoaspirat **caractérisée en ce que** la préparation est filtrée stérilement et comprend au moins l'une d'une fraction lipidique et d'une fraction aqueuse d'un lipoaspirat.

2. Préparation acellulaire selon la revendication 1, dans laquelle la fraction aqueuse comprend une solution de liposuccion usagée, avec ou sans anesthésique local et/ou adrénaline.

3. Préparation acellulaire selon la revendication 1 ou 2, dans laquelle la préparation comprend un ou plusieurs composants choisis parmi le groupe constitué de protéines de matrice extracellulaire structurelles et non structurelles et de peptides, de chaînes et de sous-unités desdites protéines de matrice extracellulaire; ; de lipides, de protéines associées aux lipides et de protéines liées au métabolisme des lipides choisis parmi le groupe constitué de céramides, d'apolipoprotéines, de périlipines, d'acides gras non saturés et saturés, de glycérophospholipides, d'acides lysophosphatidiques, de lysophospholipides, de monoglycérides, de diglycérides, de triglycérides et de prostaglandines ; de protéines de membrane basale et de chaînes peptidiques, et de sous-unités desdites protéines de membrane basale ; de composants spécialisés de la jonction dermoépidermique ; d'acides aminés essentiels et non essentiels ; d'antioxydants ; de trétinoïne, de la rétine déshydrogénase 1, de protéine de liaison au rétinol 4, de transthyrétine, de protéine de liaison à la vitamine D. de nicotinamide (vitamine B3), de biotinidase, de pyridoxamine, d'acide pantothénique. de sélénoprotéine P et d'acide pyroglutamique ; et de glycoprotéines et de protéoglycanes.

4. Préparation acellulaire selon la revendication 3, dans laquelle
i) lesdites protéines de matrice extracellulaire structurelles et non structurelles et les peptides, les chaînes et les sous-unités desdites protéines de matrice extracellulaire comportent un ou plusieurs composants choisis parmi le groupe constitué de collagènes I, III, IV, VI, XV, XVIII, de fibronectine, de vitronectine, d'élastine, d'hyaluronane, de décorine, de ténascine, de laminine, de lumican et de prolargine ; et/ou
ii) lesdites protéines de membrane basale et peptides, les chaînes et les sous-unités desdites protéines de membrane basale et lesdits composants de la jonction dermoépidermique comportent un ou plusieurs composants choisis parmi le groupe constitué de la kératine, de la nidogène, du versican, d'intergrines, de la périplakine et de la plectine.

5. Préparation acellulaire selon l'une quelconque des revendications 1 à 4, dans laquelle la préparation peut être obtenue par un procédé comprenant les étapes consistant à :
- éventuellement, secouer un lipoaspirat humain ;
- éliminer une fraction de tissu adipeux du lipoaspirat, ladite fraction comprenant des cellules ; et
- stériliser des fractions restantes du lipoaspirat par filtration.

6. Composition cosmétique ou pharmaceutique comprenant la préparation acellulaire selon l'une quelconque des revendications 1 à 5 et un support, un adjuvant et/ou un excipient physiologiquement acceptable.

7. Composition cosmétique ou pharmaceutique selon la revendication 6, dans laquelle la composition se présente sous la forme d'une pommade, une lotion, une crème, un gel, un hydrogel, une émulsion huile dans eau, une émulsion eau dans huile, une goutte, un spray, un liquide, une solution, une poudre, une formulation à libération lente ou prolongée, un masque facial, un patch cutané, une mousse ou une préparation moussante.

8. Composition cosmétique ou pharmaceutique selon la revendication 6 ou 7, dans laquelle la composition est prévue pour une administration topique ou une administration sous-cutanée, intradermique ou intrahypodermique.

9. Utilisation de la préparation acellulaire selon l'une quelconque des revendications 1 à 5 ou de la composition cosmétique selon l'une quelconque des revendications 6 à 8 destinées au traitement cosmétique de la peau.

10. Procédé non thérapeutique destiné au traitement cosmétique de la peau, le procédé comprenant l'étape consistant à l'application de la préparation acellulaire selon l'une des revendications 1 à 5 ou de la composition cosmétique selon l'une quelconque des revendications 6 à 8.

11. Utilisation selon la revendication 9 ou procédé selon la revendication 10, dans lesquels la peau est une peau compromise, de préférence préparée par micro-aiguillage, exfoliation et/ou resurfaçage au laser.

12. Utilisation selon la revendication 9 ou procédé selon la revendication 10, dans lesquels la peau est une peau mature.

13. Préparation acellulaire selon l'une quelconque des revendications 1 à 5 ou composition pharmaceutique selon l'une quelconque des revendications 6 à 8 pour une utilisation dans le traitement d'un état clinique de la peau.

14. Préparation acellulaire ou composition pharmaceutique pour une utilisation selon la revendication 13, dans laquelle l'état clinique de la peau est l'acné, la kératose actinique, la dermatite atopique, la dermatite de stase veineuse, l'eczéma, la dermatite de contact, le mélasma, le vitiligo, le psoriasis, la rosacée, la dermatite séborrhéique ou l'épidermolyse bulleuse.

15. Procédé de fabrication d'une composition cosmétique pour un traitement non thérapeutique de la peau comprenant la préparation acellulaire selon l'une quelconque des revendications 1 à 5.
